# EUROPEAN PATENT SPECIFICATION

(11) **EP 0 481 118 B1**
(45) Date of publication and mention of the grant of the patent: **02.08.1995**
(21) Application number: 90120051.9
(22) Date of filing: 19.10.1990
(51) Int. Cl.: C07D 257/04

(54) **A method for producing butyl 3'-(1H-tetrazol-5-yl) oxanilate**
Verfahren zur Herstellung von Butyl-3'-(1H-tetrazol-5-yl)-oxanilat
Procédé de préparation de 3'-(1H-tétrazol-5-yl)oxanilate de butyle

(43) Date of publication of application: 22.04.1992
(73) Proprietor: Wakamoto Pharmaceutical Co., Ltd., Chuo-ku, Tokyo 103 (JP)
(72) Inventor: Horio, Yoshihiro, Hatano-shi, Kanagawa-ken (JP); Ootake, Yasuhiro, Minami-Ashigara-shi, Kanagawa-ken (JP); Sawaki, Shohei, Naka-gun, Kanagawa-ken (JP); Goto, Masayoshi, Isehara-shi, Kanagawa-ken (JP)
(74) Representative: VOSSIUS & PARTNER

(56) References cited:
- EP-A- 0 256 507
- EP-A- 0 388 967
- GB-A- 2 170 403

## Description

The present invention relates to an industrially advantageous method for producing butyl 3'-(1H-tetrazol-5-yl) oxanilate (hereinafter referred to as "TAZANOLAST") which is an excellent antiallergic agent.

The TAZANOLAST may be produced according to the method described in claim 2 of JP-B-59-1705 , that is, the method for producing aminophenyl tetrazole derivatives represented by the formula: 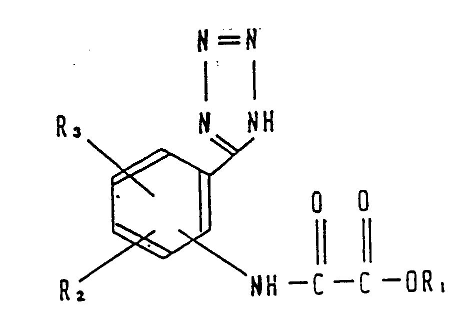
(in the formula R₁ is a lower alkyl group, R₂ and R₃ may be the same or different from each other and each is a hydrogen atom, lower alkyl group, lower alkoxy group or halogen atom) and the salts thereof with a pharmaceutically suitable ion, characterized in that a compound represented by the formula: 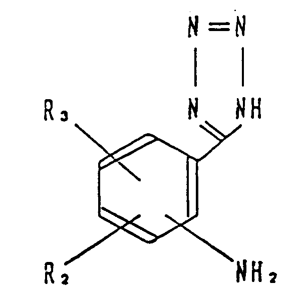
(in the formula R₂ and R₃ are as defined above) is reacted with a compound represented by the formula: 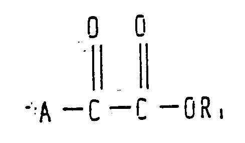
(in the formula A is a halogen atom or lower alkoxy group and R₁ is a lower alkyl group), and, if desired, salts may be made with a pharmaceutically suitable ion.

More specifically, TAZANOLAST may be easily produced by reacting 3-(1H-tetrazol-5-yl)aniline with an oxalic acid derivative.

In the case where butyl oxalyl chloride is used as an oxalic acid derivative, the reaction must be carried out in an inactive organic solvent, in the presence of a base such as triethyl amine or pyridine at room temperature or below.

On the other hand, in the case where dibutyl oxalate is used, the reaction may be carried out in an inactive organic solvent. However, this makes the reaction slow and the yield of product low. Therefore, the starting material is used as a solvent and reacted with each other by heating them to 100 ∼ 180°C, and then purified by recrystallization to obtain TAZANOLAST.

However, these methods have many industrial disadvantages and are accordingly, not practical methods for producing TAZANOLAST. For example, in the case where butyl oxalyl chloride is used, there are many difficulties in obtaining the starting material and in maintaining the anhydrous conditions in the reaction. In the case where dibutyl oxalate is used, high coloring occurs because it requires high temperature reaction conditions, such as 100 ∼ 180 °C, and thereby a troublesome process is required for decoloring. A reaction at a high temperature of 100 °C or higher is inadequate for maintaining the stability of the tetrazole group.

Further, in both of the above reactions, TAZANOLAST produced contains 3'-(1H-tetrazol-5-yl)oxanilic acid as a byproduct which is produced by hydrolyzing the butyl ester, which makes TAZANOLAST inadequate for use as a pharmaceutical. That is, these reactions have a disadvantage, that it is difficult to completely eliminate 3'-(1H-tetrazol-5-yl)oxanilic acid with the conventional purification processes such as recrystallization and column chromatography.

In EP-A 388 967 a reaction is described for preparing TAZANOLAST wherein 5-(3-aminophenyl)tetrazole is reacted with n-butyl oxalate at a temperature of 165-175°C in the absence of an acid.

Accordingly, it is an object of the present invention to solve the above problems and to provide an industrially advantageous method for producing pharmaceutically acceptable TAZANOLAST having high purity.

In accordance with the present invention, the foregoing object is achieved by adding an acid as a reaction accelerator to a liquid mixture of 3-(1H-tetrazol-5-yl)aniline and dibutyl oxalate. Incidentally, when an adsorbent, especially activated alumina is used 3'-(1H-tetrazol-5-yl)oxanilic acid is easily and completely eliminated, which previously had been difficult to do.

The present invention relates to a method for producing TAZANOLAST comprising reacting 3-(1H-tetrazol-5-yl)aniline (I) with dibutyl oxalate (II) in the presence of an acid. More specifically, the present method comprises adding an acid as a reaction accelerator to a liquid mixture of the (I) and (II), and preferebly reacting them at a temperature which is not relatively high (100 °C or lower). The present invention further comprises purifying the reaction product using an adsorbent, especially activated alumina.

In the present invention, the amount of dibutyl oxalate (II) used is not limited because it behaves as a reaction solvent. But from several viewpoints, it is preferably that it is used in an amount of five to ten times that of 3-(1H-tetrazol-5-yl)aniline.

The acid added as a reaction accelerator includes organic acids such as formic acid, acetic acid, oxalic acid, malonic acid, pyruvic acid, oxalacetic acid. Acetic acid is preferred.

The acid is used in an amount of preferably 0.05 to equal mol, more preferably 0.3 to 0.4 mol per 3-(1H-tetrazol-5-yl) aniline (I).

Reaction temperature and reaction time may be chosen suitably. But, the reaction proceeds advantageously, at a temperature between 80 to 100°C (preferably 85 to 90 °C) in view of the stability of a tetrazole group, for 12 to 20 hours, preferably 15∼18 hours.

Another feature of the present invention resides in a purification method using a specific adsorbent such as activated alumina.

The preferable method using activated alumina will be detailed below.

The purification method using activated alumina may be made either after producing crude TAZANOLAST product from the reaction liquid mixture or directly in the liquid mixture. Either of batch process or column process may be used depending on the necessity.

Crude TAZANOLAST can be obtained by crystallizing it from the reaction liquid mixture with an organic solvent in which TAZANOLAST is insoluble, such as n -hexane, ethyl ether, i-propyl ether, benzene or toluene. Crude TAZANOLAST can also be obtained by a method in which an aqueous sodium bicarbonate solution is added to the reaction liquid mixture to convert it to a sodium salt, and the salt is transferred to the water phase, and after eliminating dibutyl oxalate, it is crystallized by acidifying the phase with hydrochloric acid. Both methods may be considered as industrially advantageous methods because dibutyl oxalate can be recovered.

Any kinds of the activated aluminas can be used.

The one for purification by adsorption is preferable. In particular, γ -alumina is most preferable. The activated alumina has an ignition loss of preferably about 1 to 6%, and more preferably 3 to 5%.

The amounts of the activated alumina vary depending on the purification method adopted. However, the activated alumina is used preferably in an amount of 0.6 to 3.0 times and more preferably in an amount of 0.8 to equal times that of the crude product when a crude product is produced. When TAZANOLAST is directly purified from the reaction liquid mixture, the activated alumina is used preferably in an amount of 2.0 to 5.0 times and more preferably in an amount of 2.5 to 3.0 times that of the (I) as a starting material.

Any kinds of the organic solvents used in the purification can be used as long as they may be generally used for activated alumina. However, in view of the stability and solubility of TAZANOLAST, ketones such as acetone, methyl ethyl ketone and esters such as methyl acetate and ethyl acetate are preferable, and ethyl acetate is more preferable.

When the crude TAZANOLAST product is purificd, the organic solvent is used preferably in an amount of 20 to 60 times and more preferably in an amount of 30 to 50 times that of the crude TAZANOLAST product. When TAZANOLAST is directly purified from the reaction liquid mixture, the organic solvent is used preferably in an amount of 30 to 100 times, more preferably in an amount of 50 to 60 times that of the (I) as a starting material.

The TAZANOLAST solution obtained by the above method may be concentraed and recrystallized as usually carried out, to produce high purity TAZANOLAST in high yield.

Any kinds of the organic solvents used in the recrystallization can be used. Ethyl acetate which is used in the purification process using activated alumina is preferable in view of stability of TAZANOLAST and cost of production. The organic solvent is used preferably in an amount of six to ten times, and more preferably in an amount of eight to ten times that of TAZANOLAST purified.

According to the present invention, TAZANOLAST having purity which is fully acceptable for a pharmaceutical can be produced industrially advantageously, reproducibly and in high yield.

The present invention will be hereinafter described in more detail with reference to the following working Examples.

### Example 1

5 g (31 mmol) of 3-(1H-tetrazol-5-yl)aniline was suspended in 25 ml (123 mmol) of dibutyl oxalate and 0.65 ml (11.4 mmol) of acetic acid was added to the suspension and stirred at an internal temperature of 85 to 90 °C for 17 hours. TAZANOLAST in the reaction mixture liquid mixture was determined by liquid chromatography. The yield of TAZANOLAST was 98.2%.

This example was repeated except for using various acids in place of acetic acid, The results are shown in Table 1.

**Table 1**

| Acid | Amount used (mmol) | TAZANOLAST (%) |
|---|---|---|
| ... | ... | 48.5 |
| formic acid | 0.5 | 67.3 |
| malonic acid | 0.25 | 64.7 |
| pyruvic acid | 0.5 | 95.2 |
| oxalacetic acid | 0.25 | 84.5 |
| oxalic acid | 0.5 | 92.5 |
| acetic acid | 0.5 | 88.0 |

### Example 2

20 kg (124 mol) of 3-(1H-tetrazol-5-yl)aniline was added to 100 ℓ (495 mol) of dibutyl oxalate and 2.6 ℓ (45.5 mol) of acetic acid was added thereto, and stirred at an internal temperature of 85 to 90°C for 17 hours. After completing the reaction, the reaction liquid mixture was cooled and an aqueous sodium bicarbonate solution was added to the mixture, which was stirred at a room temperature for 30 minutes to dissolve it. The reaction mixture was left to stand and an organic layer was eliminated by a separating funnel . The aqueous layer was acidified with hydrochloric acid and crude TAZANOLAST was crystallized. The crude TAZANOLAST was suspended in ethyl acetate, to which 30 kg of activated alumina was added and stirred at a room temperature. Then, the activated alumina was filtered off under a reduced pressure, and washed with ethyl acetate. The filtrate and wash liquid was combined and concentrated under a reduced pressure and recrystallized to produce 26 kg of TAZANOLAST.
Yield: 72.4%
m.p.: 157 ∼ 158°C

| Elemental analysis (C₁₃H₁₅N₅O₃) | | | |
|---|---|---|---|
| | C | H | N |
| Calcd.(%) | 53.97 | 5.23 | 24.21 |
| Found (%) | 53.65 | 5.39 | 24.13 |

I.R. nujol max ν(cm ⁻¹); 3350, 1730, 1500, 1290, 1240, 1080, 750
N.M.R. (DMSO-d₆ ) δ : 0.95(t,3H), 1.50(m,2H), 1.80(m,2H), 4.3 3(t,2H), 7.60(t,1H), 7.90(m,2H), 8.65(m,1H), 11.08(s,1H)
Mass: m/z 289 [M ⁻ ]

### Example 3

500 ℓ of ethyl acetate was added to the reaction liquid mixture, which had been obtained in a similar way to Example 2, to produce a suspension. 50 kg of activated alumina was added to the suspension and stirred at a room temperature. Then, the activated alumina was filtered off under a reduced pressure, washed with ethyl acetate, and the filtrate and wash liquid were combined and concentrated under a reduced pressure. The residue was crystallized with n-hexane and the crystallized TAZANOLAST was filtered under a reduced pressure and recrystallized, to produce 27 kg of TAZANOLAST. (Yield) 75.2%.

The results obtained by instrumental analysis correspond to those of Example 2.

## Claims

1. A method for producing butyl 3'-(1H-tetrazol-5-yl) oxanilate comprising reacting 3-(1H-tetrazol-5-yl)aniline with dibutyl oxalate in the presence of an acid, selected from formic acid, malonic acid, pyruvic acid, oxalacetic acid, oxalic acid and acetic acid wherein said dibutyl oxalate is used in an amount of five to ten times that of said 3-(1H-tetrazol-5-yl)aniline and said acid is used in an amount of 0.05 to 1.0 mol per 3-(1H-tetrazol-5-yl)aniline, whereby the reaction is carried out at a temperature of 80 to 100 °C. for 12 to 20 hours

2. The method of claim 1 wherein the reaction product is purified with an adsorbent after the reaction.

3. The method of claim 2 wherein said adsorbent is activated alumina.

## Patentansprüche

1. Verfahren zur Herstellung von 3'-(1H-Tetrazol-5-yl)oxanilsäurebutylester, umfassend die Umsetzung von 3-(1H-Tetrazol-5-yl)anilin mit Oxalsäuredibutylester in Gegenwart einer Säure, ausgewählt aus Ameisensäure, Malonsäure, Brenztraubensäure, Oxalessigsäure, Oxalsäure und Essigsäure, wobei der Oxalsäuredibutylester in einer fünf- bis zehnfachen Menge vom 3-(1H-Tetrazol-5-yl)anilin und die Säure in einer Menge von 0,05 bis 1,0 Mol pro 3-(1H-Tetrazol-5-yl)anilin verwendet wird, und wobei die Umsetzung 12 bis 20 Stunden lang bei einer Temperatur von 80 bis 100°C durchgeführt wird.

2. Verfahren gemäß Anspruch 1, wobei das Reaktionsprodukt nach der Umsetzung mit einem Adsorbens gereinigt wird.

3. Verfahren gemäß Anspruch 2, wobei das Adsorbens aktiviertes Aluminiumoxid ist.

## Revendications

1. Procédé de préparation de 3'-(1H-tétrazol-5-yl)oxanilate de butyle, comprenant l'étape consistant à faire réagir de la 3-(1H-tétrazol-5-yl)aniline avec de l'oxalate de dibutyle en présence d'un acide choisi parmi l'acide formique, l'acide malonique, l'acide pyruvique, l'acide oxalacétique, l'acide oxalique et l'acide acétique, dans lequel on utilise ledit oxalate de dibutyle en une quantité de cinq à dix fois celle de ladite 3-(1H-tétrazol-5-yl)aniline et on utilise ledit acide en une quantité de 0,05 à 1,0 mole par mole de 3-(1H-tétrazol-5-yl)aniline, la réaction étant effectuée à une température de 80 à 100°C, pendant 12 à 20 heures.

2. Procédé selon la revendication 1, dans lequel, après la réaction, on purifie le produit de la réaction à l'aide d'un adsorbant.

3. Procédé selon la revendication 2, dans lequel ledit adsorbant est de l'alumine activée.
